# EUROPÄISCHE PATENTSCHRIFT

(11) **EP 1 009 470 B1**
(45) Veröffentlichungstag und Bekanntmachung des Hinweises auf die Patenterteilung: **28.02.2007**
(21) Anmeldenummer: 98947481.2
(22) Anmeldetag: 29.08.1998
(51) Int. Cl.: A61M 25/10, A61F 2/06

(54) **BALLONKATHETER**
BALLOON CATHETER
CATHETER A BALLONNET

(30) Priorität: 06.09.1997 DE 19739086
(43) Veröffentlichungstag der Anmeldung: 21.06.2000
(73) Patentinhaber: Völker, Wolfram, 69469 Weinheim (DE); Siess, Thorsten, 52146 Würselen (DE); Reul, Helmut, Prof. Dr., 52353 Düren (DE)
(72) Erfinder: Völker, Wolfram, 69469 Weinheim (DE); Siess, Thorsten, 52146 Würselen (DE); Reul, Helmut, Prof. Dr., 52353 Düren (DE)
(74) Vertreter: Selting, Günther
(86) Internationale Anmeldenummer: PCT/EP1998/005506
(87) Internationale Veröffentlichungsnummer: WO 1999/012601

(56) Entgegenhaltungen:
- WO-A-96/38109
- US-A- 4 423 725
- US-A- 5 304 135
- US-A- 5 409 495

## Beschreibung

Die Erfindung betrifft einen Ballonkatheter mit einem Katheterschlauch, auf dem mindestens zwei durch Innendruck aufweitbare Ballons sitzen.

Ballonkatheter werden in Blutgefäße eingeführt, um Stenosen, d.h. durch Ablagerungen verursachte Verengungen, durch Dilatation zu beseitigen. Ein Ballonkatheter weist einen Hochdruckballon auf, der beim Einführen des Katheters im gefalteten Zustand ist und nach Erreichen der Stenose durch Füllen mit einer Flüssigkeit aufgeweitet wird. Hierbei wird ein relativ hoher Druck in der Größenordnung von ca. 3-20 hPa aufgebracht. Der Hochdruckballon drückt dadurch die Verengung auseinander, um den dortigen Öffnungsquerschnitt bleibend zu erweitern.

Ferner ist es bekannt, um den Ballon herum einen rohrförmigen Koronarstent aus Metall anzuordnen, der durch das Aufweiten des Ballons ebenfalls aufgeweitet wird und in der Arterie verbleibt, nachdem der Katheter herausgezogen wurde. Bei der Implantation von Koronarstents kann es durch den Ballon zu Einrissen der angrenzenden Gefäßwand kommen (Peristentdissektion). Dies ist dadurch bedingt, daß der Ballon nicht nur im Stentbereich, sondern auch außerhalb des Stents aufgeweitet wird und an der Übergangsstelle mit hohem Druck gegen die Gefäßwand drückt. Im Falle einer Peristentdissektion muß ggf. ein weiterer Stent in Nachbarschaft des ursprünglich eingesetzten Stents implantiert werden, um die Dissektion abzudecken. Alternativ kann versucht werden, durch Vermeidung eines hohen Ballondrucks und Unterdimensionierung des Stents eine Überdehnung im Peristentbereich zu vermeiden. Diese Methode birgt allerdings die Gefahr, daß der Stent nur unzureichend expandiert wird. Zudem besteht die Gefahr des Frühverschlusses oder der Restenose.

Ein weiteres Problem bei Ballonkathetern besteht darin, daß der langgestreckte Ballon, der mit hohem Druck aufgeweitet wird, die Arterie über die gesamte Ballonlänge in eine gerade Form streckt, selbst wenn der natürliche Verlauf der Arterie an dieser Stelle gekrümmt ist.

Aus US 4 748 981 ist ein Ballonkatheter bekannt, der zwei ineinander angeordnete Ballons enthält. Dieser Ballonkatheter dient dazu, zwei Dilatationsvorgänge an unterschiedlichen Stellen der Arterie nacheinander mit ggf. unterschiedlichen Durchmessern durchführen zu können, ohne einen Katheterwechsel durchführen zu müssen.

Ein mit zwei Ballons versehener Ballonkatheter ist bekannt aus DE 195 26 784 A1 und DE 297 04 280 U1. Dieser Ballonkatheter hat einen ersten Ballon, der zum Setzen eines Stents benutzt werden kann, und einen im Abstand hiervon an dem Katheterschlauch angebrachten zweiten Ballon, der als Dilatationsballon verwendet wird. Zuerst wird der zweite Ballon in den Bereich der Stenose gebracht und aufgeweitet. Danach wird der Katheter vorgeschoben, so dass der erste Ballon, der einen Stent trägt, in der Stenose plaziert wird. Dabei kann der erste Ballon als Hochdruckballon und der zweite Ballon als Niederdruckballon ausgebildet sein.

Der Oberbegriff des Patentanspruchs 1 geht aus von einem Ballonkatheter nach US-A-5, 409, 495. Dieser weist einen inneren Ballon und zwei an die Enden des inneren Ballons anschließende äußere Ballons auf. Der innere Ballon und die äußeren Ballons sind mit unterschiedlichen Lumen des Katheterschlauchs verbunden, so dass sie unterschiedlich stark insuffliert werden können. Die äußeren Ballons bestehen aus einem Material, das hohem Druck standhält, während der innere Ballon aus einem weicheren Material besteht. Nachdem der innere Ballon aufgeweitet wurde, werden die äußeren Ballons insuffliert, damit sie die Expansion des weicheren inneren Ballons in Längsrichtung begrenzen.

Der Erfindung liegt die Aufgabe zugrunde, einen Ballonkatheter zu schaffen, mit dem Dessektionen der Gefäßwand vermieden werden und der daher eine schonende Gefäßdilatation ermöglicht.

Der erfindungsgemäße Ballonkatheter ist durch die Merkmale des Patentanspruchs 1 bezeichnet.

Der erfindungsgemäße Ballonkatheter weist außer dem ersten Ballon mindestens einen unmittelbar daran angrenzenden zweiten Ballon auf. Der erste und der zweite Ballon bilden im aufgeweiteten Zustand eine in Längsrichtung nicht durch Einschnürungen unterbrochene Abstützung, d.h. sie schließen unmittelbar aneinander an, wobei beide Ballons im aufgeweiteten Zustand entweder gleiche Durchmesser haben oder der erste Ballon einen Durchmesser hat, der etwas größer ist als der zweite Ballon. Keinesfalls ist der Durchmesser des ersten Ballons geringer als derjenige des zweiten Ballons. Beide

Ballons bilden eine sich über die gesamte Länge des Ballonbereichs erstreckende ununterbrochene Stützfläche, die an der Gefäßwand zur Anlage gebracht werden kann. Während der erste Ballon mit hohem Druck in der Größenordnung von 15 bar beaufschlagt wird, wird der zweite Ballon mit einem geringeren Druck aufgeweitet, in der Größenordnung von etwa 6 hPa*.* Dies hat zur Folge, daß über die gesamte Ballonlänge ein Ballonabschnitt zur Verfügung steht, der prall aufgeweitet werden kann, und daran angrenzend mindestens ein weiterer Ballonabschnitt, der weniger hart aufgeweitet wird. Der gesamte Ballonbereich hat im aufgeweiteten Zustand eine gestufte Härte oder Steifigkeit, so daß ein abrupter Übergang zu dem harten Ballonabschnitt vermieden wird. Dies hat zunächst den Vorteil, daß nach dem Einführen des Ballonbereichs zunächst der zweite Ballon aufgeweitet werden kann, um den Ballonbereich in der Arterie zu fixieren. Bei der nachfolgenden Aufweitung des ersten Ballons kann dieser nicht von der Stenose abgleiten und sich unbeabsichtigt in Längsrichtung verschieben. Ein weiterer Vorteil besteht darin, daß der zweite Ballon eine gewisse Verformbarkeit und Biegbarkeit hat, so daß er sich an einen gekrümmten Verlauf der Arterie besser anpassen kann. Gegenüber den bisher üblichen Ballonkathetern kann der Hochdruckballon mit kürzerer Länge ausgebildet werden, so daß er gezielt nur auf den Bereich der Stenose einwirkt.

Die auch als Hochdruckballon und Niederdruckballon bezeichneten Ballons werden im Betrieb mit höherem Druck bzw. niedrigerem Druck aufgeblasen. Dies bedeutet jedoch nicht, daß diese Ballons aus unterschiedlichen Materialien bestehen müssen oder unterschiedliche

Druckbelastbarkeiten haben. Grundsätzlich können beide Ballontypen derart ausgebildet sein, daß sie mit hohem Druck von bis zu 25 hPa betrieben werden können.
Durch Insufflation des Hochdruckballons kann ein Druck auf den Niederdruckballon ausgeübt werden, wodurch es erforderlich ist, Flüssigkeit aus dem Niederdruckballon abzulassen. Der Niederdruckballon ist daher vorzugsweise an eine Druckquelle angeschlossen, die bei Überschreiten des Niederdrucks eine Rückströmung des in den Niederdruckballon eingeführten Druckfluids zur Druckentlastung ermöglicht.

Der erfindungsgemäße Ballonkatheter kann mit Vorteil insbesondere auch für die Stentimplantation benutzt werden. Hierbei erstreckt sich der Stent in Längsrichtung bis in den Bereich des zweiten Ballons hinein. Der Stent wird also in unterschiedlichen Bereichen mit unterschiedlicher Kraft aufgeweitet und gegen die Gefäßwand gedrückt. Am Ende des Stents wirkt nur die relativ kleine Aufweitungskraft des Niederdruckballons, so daß der über den Stent überstehende Bereich des Niederdruckballons die Gefahr von Peristentdissektionen verringert. Der äußere weiche Ballon dient unter anderem zur Fixierung und Stabilisierung des Stents, so daß dieser nicht von dem inneren harten Ballon abgleitet. Es ist alternativ auch möglich, den Stent so anzuordnen, daß er nur über dem ersten Ballon liegt und sich nicht über den zweiten Ballon erstreckt.

Der Hochdruckballon ist vorzugsweise - in Längsrichtung des Katheterschlauchs - mittig in dem zweiten Ballon angeordnet, wobei die Länge der beiden Bereiche des zweiten Ballons in der Summe mindestens so groß ist wie die Länge des ersten Ballons. Es ist aber auch möglich, je einen zweiten Ballon auf jeder Seite des ersten Ballons anzuordnen. Wichtig ist, daß der erste Ballon und der zweite Ballon durch unterschiedliche Lumina des Katheterschlauchs getrennt aufgeblasen werden können, wobei sie im Betriebszustand beide aufgeblasen sind. Bei der Stentexpansion wird zuerst der Niederdruckballon aufgeweitet. Hierbei wird ein relativ geringes Trauma auf das normale Gefäßareal außerhalb des Stents ausgeübt. Die geringen Expansionsdrücke ≤ 6 hPa im zweiten Ballon genügen für eine optimale Expansion des Stents in den gesunden Gefäßabschnitten und den Peristentbereichen. Anschließend erfolgt dann das Aufweiten des ersten Ballons mit höherem Druck. Es erfolgt so eine gezielte Überdehnung im Stenosebereich innerhalb des Stents, damit dort eine maximale Expansion erreicht werden kann.
Im folgenden werden unter Bezugnahme auf die Zeichnungen Ausführungsbeispiele der Erfindung näher erläutert.

Es zeigen:
- Fig. 1: einen schematischen Längsschnitt durch eine erste Ausführungsform des Ballonkatheters,
- Fig. 2: einen Querschnitt durch den Katheterschlauch entlang der Linie II-II von Fig. 1,
- Fig. 3: einen Längsschnitt durch eine zweite Ausführungsform des Ballonkatheters und
- Fig. 4: einen Längsschnitt durch eine dritte Ausführungsform.

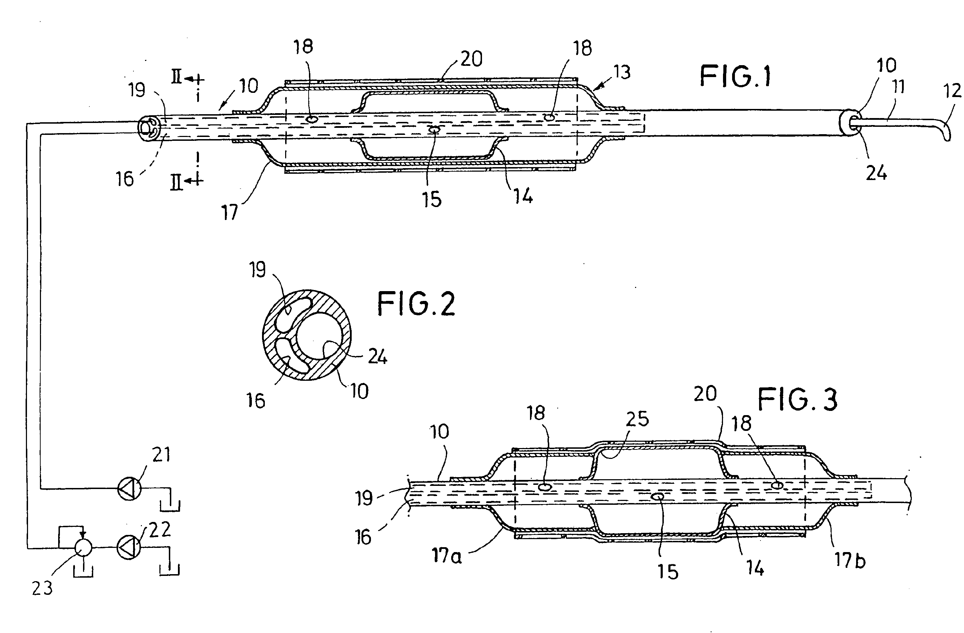

Der in Fig. 1 dargestellte Ballonkatheter weist einen langgestreckten Katheterschlauch 10 auf, der über einen Führungsdraht 11 geschoben werden kann. Vor dem Verlegen des Katheters wird zuerst der Führungsdraht 11 in die Arterie eingeschoben. Zur Wegfindung weist der Führungsdraht 11 eine weichflexible und vorbiegsame Spitze 12 auf. Wenn der Führungsdraht verlegt worden ist, wird der Katheterschlauch darübergeschoben, bis der Ballonabschnitt 13 die Stenose erreicht hat. Im Ballonbereich 13 befindet sich ein Hochdruckballon 14, der den Katheterschlauch 10 ringförmig umgibt. Das Innere des Hochdruckballons 14 steht über eine Öffnung 15 mit einem Hochdrucklumen 16 des Katheterschlauchs (Fig. 2) in Verbindung. Der Hochdruckballon 14 hat im aufgeweiteten Zustand im wesentlichen eine zylindrische Form bei einer Länge von etwa 10 mm (5-30 mm) und einem Durchmesser von etwa 2,5-6,0 mm. Die Ballonhaut hat zwar eine gewisse Elastizität, jedoch ist sie nicht hochelastisch. Im nicht-aufgeweiteten Zustand ist die Ballonhaut um den Katheterschlauch 10 herumgefaltet.

Der Hochdruckballon 14 ist von einem Niederdruckballon 17 umgeben, der bei diesem Ausführungsbeispiel den gleichen Durchmesser hat wie der Hochdruckballon 14, jedoch eine größere Länge. Die Länge des Niederdruckballons 17 beträgt etwa das Doppelte der Länge des Hochdruckballons. Auch der Niederdruckballon besteht aus einem Material von gewisser Elastizität, das jedoch nicht hochelastisch ist. Im nicht-aufgeweiteten Zustand ist der Niederdruckballon 17 ebenfalls um den Katheterschlauch 10 herumgefaltet. Der Innenraum des Niederdruckballons 17 steht über Öffnungen 18 mit einem Drucklumen 19 (Fig. 2) des Katheterschlauchs 10 in Verbindung.

Der Niederdruckballon 17 ist von einem rohrförmigen Stent 20 umgeben, welcher radial aufgeweitet werden kann. Der Stent 20 besteht in bekannter Weise aus metallischem Streckmaterial. Im Ursprungszustand umschließt der Stent 20 die um den Katheterschlauch. 10 gefalteten Ballons 14,17 eng, so daß er zusammen mit dem Katheterschlauch in das Blutgefäß eingeführt werden kann. Dieser Zustand ist in der Zeichnung nicht dargestellt. Danach wird zuerst der Niederdruckballon 17 aufgeweitet, indem eine Flüssigkeit von einer Druckquelle 22 in das Niederdrucklumen 19 hineingedrückt wird. Dadurch wird der Niederdruckballon 17 mit relativ niedrigem Druck von ca. 6 hPa aufgeweitet, wobei gleichzeitig der Stent 20 aufgedrückt wird. Danach wird Druckflüssigkeit von einer Hochdruckquelle 21, die einen Druck von bis zu 25 hPa liefert, in das Hochdrucklumen 16 hineingedrückt, wodurch der Hochdruckballon 14 aufgeweitet wird. Da hierbei Druckflüssigkeit aus dem Niederdruckballon 17 verdrängt wird, ist in Verbindung mit der Druckquelle 22 ein Druckregelventil 23 vorgesehen, das den eingestellten Niederdruck (6 hPa) beibehält und verdrängte Druckflüssigkeit abläßt.

Die beiden Enden des rohrförmigen Stents 20 befinden sich in denjenigen Bereichen, die nur von dem Niederdruckballon 17 eingenommen werden. An den Endkanten des Stents 20 wirkt der Niederdruckballon 17 infolge seiner geringeren Kraft schonend auf die Gefäßwand ein, so daß die Gefahr von Peristentdissektionen verringert ist.

Wie Fig. 2 zeigt, verläuft durch den Katheterschlauch 10 ein kreisförmiges Lumen 24 für den Führungsdraht 11. Das Hochdrucklumen 16 und das Niederdrucklumen 19 verteilen sich jeweils nierenförmig um das größere Lumen 24.

Während bei dem Ausführungsbeispiel nach Fig. 1 der Hochdruckballon 14 im aufgeweiteten Zustand den gleichen Durchmesser hat wie der Niederdruckballon 17, zeigt Fig. 3 ein Ausführungsbeispiel, bei dem der Durchmesser des Hochdruckballons 14 größer ist. Hierbei ist zu jeder Seite des Hochdruckballons 14 ein Niederdruckballon 17a,17b angeordnet. An der Übergangsstelle 25 ist die Haut des Niederdruckballons 17 mit derjenigen des Hochdruckballons 14 verklebt oder verschweißt. Der Hochdruckballon 14 hat bei diesem Ausführungsbeispiel einen Expansionsdurchmesser von 4 mm, während die Niederdruckballons 17a,17b einen Expansionsdurchmesser von 3,5 mm haben. Dies führt dazu, daß der Stent 20 in der in Fig. 3 dargestellten Weise verformt wird.

Alternativ können die Niederdruckballons 17a,17b in Fig. 3 wie in Fig. 1 zu einem Ballon 17 zusammengefaßt sein. Der größere Hochdruckballon 14 aus Fig. 3 bewirkt bei voller Druckbeaufschlagung eine Weitung des flexiblen Niederdruckballons 17, so daß es zu einer ähnlichen Aufweitung, wie in Fig. 3 dargestellt, im stenosierten Bereich kommt.

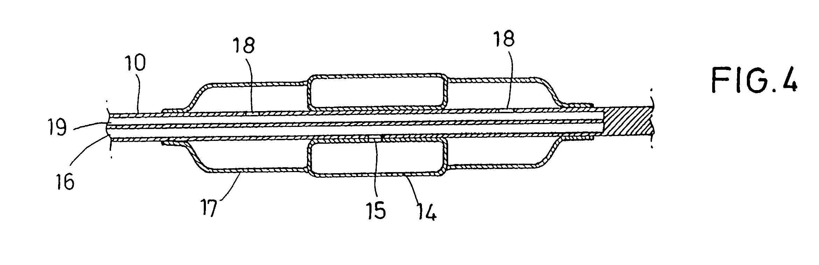

In Fig. 4 ist ein Ausführungsbeispiel dargestellt, bei dem der Hochdruckballon 14 als Ringballon ausgebildet ist, der in die Umfangswand des Niederdruckballons 17 integriert ist.

Der erfindungsgemäße Ballonkatheter kann sowohl für die Implantation eines Stents benutzt werden als auch für die einfache Gefäßdilatation (ohne Stent). Der erste und der zweite Ballon schließen unmittelbar aneinander an und haben jeweils eine gemeinsame Trennwand.

## Patentansprüche

1. Ballonkatheterset mit einem Katheterschlauch (10), auf dem ein durch Innendruck aufweitbarer erster Ballon (14) und mindestens ein separat aufweitbarer zweiter Ballon (17) vorgesehen sind, wobei beide Ballons unmittelbar aneinander anschließen oder der eine Ballon beidendig des anderen Ballons - diesen übergreifend - angeordnet ist, und mit Druckquellen (21,22), an die die Ballons (14,17) angeschlossen sind, und die es ermöglichen, beide Ballons gleichzeitig mit unterschiedlichen Drücken zu versorgen, **dadurch gekennzeichnet, dass** beide Ballons im aufgeweiteten Zustand eine in Längsrichtung ununterbrochene Stützfläche von gestufter Härte bilden,
dass der zweite Ballon (17) ein Niederdruckballon ist und
dass die Druckquelle (22), an die der zweite Ballon (17) angeschlossen ist, derart ausgebildet ist, dass sie bei Überschreiten des vorbestimmten Niederdrucks infolge einer durch Druckbeaufschlagung des ersten Ballons im zweiten Ballon hervorgerufenen Druckerhöhung eine Rückströmung des in den Niederdruckballon (17) eingeführten Druckfluids zur Druckentlastung ermöglicht.

2. Ballonkatheterset nach Anspruch 1, **dadurch gekennzeichnet, dass** der erste Ballon (14) an eine Druckquelle (21) von höherem Druck und der zweite Ballon (17) an eine Druckquelle von geringerem Druck angeschlossen ist.

3. Ballonkatheterset nach Anspruch 1 oder 2, **dadurch gekennzeichnet, dass** ein zweiter Ballon (17a,17b) an jedem der beiden Enden des ersten Ballons (14) angeordnet ist.

4. Ballonkatheterset nach einem der Ansprüche 1-3, **dadurch gekennzeichnet, dass** ein rohrförmiger aufweitbarer Stent (20) vorgesehen ist, der den ersten Ballon (14) umgibt und dessen Enden sich jeweils auf einem zweiten Ballon (17;17a,17b) befinden.

## Claims

1. A balloon catheter set comprising a catheter tube (10) having provided thereon a first balloon (14) to be expanded by internal pressure and at least one second balloon (17) to be expanded separately, both balloons directly joining each other or one of the balloons being arranged on both sides of the other balloon while overlapping the same, and comprising pressure sources (21,22) having the balloons (14,17) connected thereto and being adapted to supply different pressures to both balloons simultaneously,
**characterized in**
**that** both balloons in their expanded condition form a longitudinally continuous support face of gradually varying hardness, that the second balloon (17) is a low-pressure balloon and that the pressure source (22) which has the second balloon (17) connected thereto is so configured that, when the predetermined low pressure is exceeded, a pressure increase generated in the second balloon by pressurizing the first balloon will cause the pressurized fluid introduced into the low-pressure balloon (17) to flow back, for pressure relief.

2. The balloon catheter set according to claim 1, **characterized in that** the first balloon (14) is connected to a pressure source (21) supplying higher pressure and the second balloon (17) is connected to a pressure source supplying lower pressure.

3. The balloon catheter set according to claim 1 or 2, **characterized in that** both ends of the first balloon (14) have a second balloon (17a,17b) arranged thereon.

4. The balloon catheter set according to any one of claims 1-3, **characterized by** a tubular expandable stent (20) which surrounds the first balloon (14) and has its ends respectively arranged on a second balloon (17; 17,17b).

## Revendications

1. Kit de cathéter à ballonnets comprenant un flexible de cathéter (10) sur lequel sont prévus un premier ballonnet (14) pouvant être dilaté par la pression intérieure et au moins un deuxième ballonnet (17) pouvant être dilaté de manière séparée, les deux ballonnets étant directement raccordés l'un à l'autre ou le premier ballonnet étant disposé aux deux extrémités de l'autre ballonnet en l'enveloppant, et comprenant des sources de pression (21, 22) auxquelles sont raccordés les ballonnets (14, 17) et qui permettent d'alimenter simultanément les deux ballonnets avec des pressions différentes, **caractérisé en ce que** les deux ballonnets, dans l'état dilaté, forment une surface d'appui ininterrompue dans la direction longitudinale ayant une dureté progressive, **en ce que** le deuxième ballonnet (17) est un ballonnet basse pression et **en ce que** la source de pression (22) à laquelle est raccordé le deuxième ballonnet (17) est configurée de telle sorte que, lorsque l'on dépasse la basse pression prédéterminée à la suite d'une augmentation de pression provoquée dans le deuxième ballonnet par la mise sous pression du premier ballonnet, elle permet un reflux du fluide sous pression introduit dans le ballonnet basse pression (17) pour la détente.

2. Kit de cathéter à ballonnets selon la revendication 1, **caractérisé en ce que** le premier ballonnet (14) est raccordé à une source de pression (21) d'une pression plus élevée et le deuxième ballonnet (17) est raccordé à une source de pression d'une pression plus faible.

3. Kit de cathéter à ballonnets selon la revendication 1 ou 2, **caractérisé en ce qu'**un deuxième ballonnet (17a, 17b) est disposé au niveau de chacune des deux extrémités du premier ballonnet (14).

4. Kit de cathéter à ballonnets selon l'une quelconque des revendications 1 à 3, **caractérisé en ce qu'**il est prévu un tuteur (stent) expansible tubulaire (20) qui entoure le premier ballonnet (14) et dont les extrémités se trouvent respectivement sur un deuxième ballonnet (17 ; 17a, 17b).
